**Europäisches Patentamt**

**European Patent Office**

**Office européen des brevets**

(19)

(11) Veröffentlichungsnummer: **0 009 580**
**B1**

(12) **EUROPÄISCHE PATENTSCHRIFT**

(45) Veröffentlichungstag der Patentschrift: **10.03.82**

(21) Anmeldenummer: **79102993.7**

(22) Anmeldetag: **16.08.79**

(51) Int. Cl.³: **G 05 D 21/00,**
**G 01 N 33/18, C 02 F 3/00**

(54) **Verfahren und Vorrichtung zur Regelung und/oder Steuerung einer biologischen Kläranlage.**

(30) Priorität: **03.10.78 DE 2843074**

(43) Veröffentlichungstag der Anmeldung:
**16.04.80 Patentblatt 80/8**

(45) Bekanntmachung des Hinweises auf die Patenterteilung:
**10.03.82 Patentblatt 82/10**

(84) Benannte Vertragsstaaten:
**BE CH DE FR GB IT LU NL SE**

(56) Entgegenhaltungen:
**DE - A - 2 056 973**
**DE - A - 2 338 004**
**DE - A - 2 617 174**
**FR - A - 1 567 182**
**US - A - 3 986 932**

(73) Patentinhaber: **BOEHRINGER MANNHEIM GMBH**
**Sandhofer Strasse 112-132 Postfach 31 01 20**
**D-6800 Mannheim 31-Waldhof (DE)**

(72) Erfinder: **Seydler, Bodo**
**Herzogstandstrasse 35**
**D-8120 Weilheim (DE)**
Erfinder: **Koch, Olaf**
**Alter Post Platz 3**
**D-8124 Seeshaupt (DE)**
Erfinder: **Schmieder, Günter**
**Barbaraweg 7**
**D-8132 Tutzing (DE)**
Erfinder: **Näher, Gotthilf, Dr.**
**Zugspitzstrasse 22**
**D-8132 Tutzing (DE)**

(74) Vertreter: **Weickmann, Heinrich, Dipl.-Ing**
**Patentanwälte Dipl.Ing.H.Weickmann et al,**
**Dipl.Phys.Dr.K.Fincke Dipl.Ing.F.A.Weickmann**
**Dipl.Chem.B.Huber, Dr.-Ing.H.Liska Möhlstrasse**
**22**
**D-8000 München 86 (DE)**

Courier Press, Leamington Spa, England.

Verfahren und Vorrichtung zur Regelung und/oder Steuerung einer biologischen Kläranlage

Die Erfindung betrifft ein Verfahren und eine Vorrichtung zur Regelung und/oder Steuerung einer biologischen Kläranlage mit einem Belebungsbecken, dem kontinuierlich Abwasser und Belebtschlamm zugeführt und aus dem Ablaufflüssigkeit abgezogen wird, und mit einer die Ablaufflüssigkeit aufnehmenden Trenneinrichtung, die den Schlamm von der Ablaufflüssigkeit trennt und in das Belebungsbecken teilweise rückführt, wobei man einen biologischen Sauerstoffbedarfswert einer Belebungsbeckenflüssigkeit enthaltenden Probeflüssigkeit mißt und der Regelung und/oder Steuerung der Kläranlage zugrundelegt.

Nach einem bekannten Verfahren dieser Art zur Regelung einer biologischen Kläranlage (DD—PS 47 779) wird offensichtlich der $BSB_5$-Wert gemessen, der die innerhalb von fünf Tagen vom Belebtschlamm verbrauchte Sauerstoffmenge bezeichnet. Der $BSB_5$-Wert ist jedoch zur Regelung einer Kläranlage wenig geeignet, da er auch von Sekundärreaktionen der Mikroorganismen des Schlamms nach erfolgtem Abwasserabbau abhängig ist. Hinzu kommt, daß dieses Verfahren diskontinuierlich arbeitet (Entnahme einzelner Proben zur $BSB_5$-Messung) und aufgrund der langen Meßzeit außerstande ist, auf sich rasch ändernde Verhältnisse im Belebungsbecken rechtzeitig zu reagieren.

Aus der US—PS 3 986 932 ist ein Verfahren zur Regelung des TS-Gehalts (Trockensubstanzgehalt des Belebtschlamms) bekannt, bei dem man den momentanen biologischen Sauerstoffbedarf pro Zeitzeit des Belebtschlamms vor dessen Rückführung in das Belebungsbecken mißt und diesen die endogene Atmung darstellenden Meßwert zur Regelung der Rückführrate des Belebtschlamms einsetzt. Nach dem bekannten Verfahren kann auch noch eine Sauerstoffbedarfsmessung in der gemeinsamen Zuleitung von Abwasser und rückgeführtem Belebtschlamm in das Belebungsbecken durchgeführt werden. Von dem bei dieser Messung erhaltenen, die Gesamt-Atmung darstellenden Meßwert wird der am Belebtschlamm gemessene Meßwert abgezogen, um hieraus die Substratatmung bestimmen zu können und in Abhängigkeit von diesem Wert die Schlammrückführungsrate zu regeln. Nachteilig an diesem Verfahren ist, daß die Messungen nur einen mittelbaren Schluß auf die Zusammensetzung der Belebungsbeckenflüssigkeit, insbesondere auf den TS-Gehalt im Belebungsbecken zulassen, was eine schnelle und exakte Einstellung eines optimalen TS-Gehaltswerts erschweren, wenn nicht gar unmöglich macht.

Aus der DE—OS 20 56 973 ist ein Verfahren zur schnellen Kontrolle von organischen Verunreinigungen bekannt, welches auch in Zusammenhang mit der Regelung einer biologischen Kläranlage und hierbei auch zur Festlegung der Rückführrate des Belebtschlamms eingesetzt werden kann. Bei diesem Verfahren wird jeweils an einer Einzelprobe, beispielsweise aus Belebungsbeckenflüssigkeit, eine Reihe von zeitlich aufeinanderfolgenden Sauerstoffbedarfsmessungen durchgeführt, um die Veränderung des Sauerstoffsverbrauchs in Abhängigkeit von der Zeit feststellen zu können. Die Probenflüssigkeit wird dabei mittels einer schrittweise angetriebenen Pumpe oder einer zwischengeschalteten Kammer mit veränderlichem Volumen taktweise bzw. in pulsierendem Strom an der eigentlichen Meßelektrode vorbeigeführt. Die bei einer derartigen Meßreihe an einer Einzelprobe ermittelten Meßwerte (Meßkurven) bedürfen noch der Auswertung, da erst die Hüllkurve dieser Meßkurven die festzustellende Veränderung des Sauerstoffsverbrauchs der Probenflüssigkeit angibt. Das aus der DE—OS 20 96 973 bekannte Verfahren bedarf daher eines relativ hohen Geräteaufwands; auch ist nicht ersichtlich, wie mit diesem Verfahren eine genaue Regelung des TS-Gehalts in einem Belebungsbecken durchgeführt werden kann.

Aus der DE—OS 23 38 004 ist ein Verfahren zur Überwachung des Sauerstoffgehalts von in einem Abwasserbecken zum nachfolgenden Ablassen, beispielsweise in einen See oder einen Fluß, gespeichertem Sekundärwasser bekannt. Bei diesem Verfahren wird dem Abwasserbecken kontinuierlich Abwasser entnommen und einer kontinuierlichen Messung des momentanen Sauerstoffbedarfs pro Zeitheit unterzogen, um hierdurch eine Anzeige der Menge der im Abwasser verbleibenden Bakteriennahrung zu erhalten.

Aufgabe der Erfindung ist es, ein Verfahren zur Regelung und/oder Steuerung einer biologischen Kläranlage bereitzustellen, das einfach und unter geringem Geräteaufwand durchzuführen ist und das auf Grund genauer Regelung des TS-Gehalts im Belebungsbecken einen störungsfreien Kläranlagenbetrieb auch bei wechselnder Abwasserzusammensetzung gewährleistet.

Diese Aufgabe wird dadurch gelöst, daß man zur Regelung des TS-Gehalts im Belebungsbecken dem Belebungsbecken kontinuierlich Belebungsbeckenflüssigkeit zur Bildung der Probenflüssigkeit entnimmt und daß man die Probenflüssigkeit während einer vorgegebenen Reaktionsdauer $\Delta t$ mit Sauerstoff im Überschuß versetzt und anschließend den momentanen biologischen Sauerstoffbedarf pro Zeitheit der Probenflüssigkeit kontinuierlich mißt, wobei man die Reaktionsdauer $\Delta t$ derart festlegt, daß der biologische Abbau des Abwassers in der Probenflüssigkeit innerhalb der Reaktionsdauer im wesentlichen beendet ist. Der so erhaltene Meßwert gibt direkt den TS-Gehalt im Belebungsbecken an unabhängig von der momentanen Zusammensetzung des Abwassers. Aus einer Änderung dieses Meßwerts kann man daher auf eine Änderung des TS-Gehalts schließen und entsprechende Maßnahmen zur Einstellung eines TS-Gehaltsollwertes ergreifen. Da bei diesem Verfahren kontinuierlich Probenflüssigkeit aus dem Belebungsbecken entnommen wird und der an dieser Probe gemessene Sauerstoffbedarfswert in der Regel nach einer halben Stunde vorliegt, reagiert das vorgeschlagene Regelverfahren rasch auf Änderungen der Zusammensetzung der Belebungsbeckenflüssigkeit. Die Messung und Auswertung eines einzigen kontinuierlich ermittelten Parameters läßt sich mit einfachen baulichen Mitteln durchführen.

Es wird vorgeschlagen, daß man eine weitere derartige Messung vornimmt, bei der die Reaktionsdauer jedoch derart festgelegt wird, daß am Ende der Reaktionsdauer die Substratatmungsrate der Probenflüssigkeit im wesentlichen maximal ist. Aus einer Änderung dieses Meßwerts kann man auf eine Änderung der Konzentration und/oder Zusammensetzung des Abwassers schließen und dementsprechend die Kläranlage nachregeln.

Wenn man, wie weiterhin vorgeschlagen wird, die Messung dann durchführt, wenn die Substratatmungsrate auf einen Wert abgefallen ist, der 1/5 bis 4/5, besser 2/5 bis 3/5, am besten der Hälfte der maximalen Substratatmungsrate entspricht, so kann man geringfügige Meßwertänderungen zur Regelung oder Steuerung des Belebtschlammgehalts und größere Änderungen des Meßsignals zur Regelung oder Steuerung des Abwasseranteils im Belebungsbecken einsetzen. Dies liegt daran, daß nur größere Konzentrations- oder Zusammensetzungsänderungen des Abwasseranteils im Belebungsbecken das erhaltene Meßergebnis beeinflussen.

Ferner wird vorgeschlagen, daß man in einer weiteren Messung mit dem dem Belebungsbecken kontinuierlich zufliessenden Abwasser kontinuierlich Probenflüssigkeit bildet und den biologischen Sauerstoffbedarf pro Zeiteinheit kontinuierlich mißt.

Die Meßwerte können ohne aufwendige Auswertung direkt zur Einstellung der Abführrate des Überschußschlamms, der Rückführrate des Belebtschlamms und der Zuführrate des Abwassers verwendet werden. Ebenso kann man in Abhängigkeit von den Meßwerten die Zuführrate einstellen, mit dem der Sauerstoff in das Belebungsbecken eingebracht wird.

Besonders einfach gestaltet sich das Regel- und/oder Steuerungsverfahren, wenn die Überschußschlammabführraten und die Abwasserzuführraten entsprechend geändert werden, wenn der gemessene Sauerstoffbedarf vorgegebene Extremwerte über- bzw. unterschreitet.

Es wird vorgeschlagen, daß man die Probenflüssigkeit bei der kontinuierlichen Messung des momentanen biologischen Sauerstoffbedarfs pro Zeiteinheit mit einer Fließgeschwindigkeit von 50 bis 200 cm/sec, vorzugsweise 110 bis 140 cm/sec, am besten 125 cm/sec durch eine nach aussen hin abgeschlossene Zuleitung und einen sich anschliessenden Durchflußkanal einer Meßzelle pumpt. Diese hohe Fließgeschwindigkeit bietet den Vorteil, daß besonders genau messende Sauerstoffmeßzellen eingesetzt werden können, welche eine hohe Anströmgeschwindigkeit der zu messenden Flüssigkeit erfordern. Auch wird hierdurch verhindert, daß sich in der Zuleitung und im Durchflußkanal Ablagerungen bilden. Schließlich wird auch der Sauerstoffaustausch zwischen aufeinanderfolgenden Volumeneinheiten aufgrund der entsprechend kurzen Laufzeit der Probenflüssigkeit durch die Zuleitung in engen Grenzen gehalten, was die Genauigkeit des Meßergebnisses weiter verbessert.

Bei einer Vorrichtung zur Durchführung des Verfahrens beträgt die Länge der Zuleitung 2 bis 10 m, vorzugsweise 4 bis 8 m, am besten 6 m. Dies gewährleistet eine ausreichende Laufzeit der Probenflüssigkeit durch die abgeschlossen Zuleitung bei den vorstehend genannten hohen Fließgeschwindigkeiten.

Durch die Ausbildung des Durchflußkanals der Meßzelle mit einem Knick in Strömungsrichtung anschließend an die Meßzelle wird verhindert, daß sich Schlamm- und Luftblasen an der Meßelektrode anlagern.

Die Erfindung wird im folgenden anhand der Zeichnung in einem Ausführungsbeispiel erläutert. Es zeigen

Fig. 1 eine schematische Darstellung der Regelung einer biologischen Kläranlage;

Fig. 2a eine erfindungsgemäße Vorrichtung zur Messung des Kurzzeit-BSB-Wertes einer Flüssigkeit;

Fig. 2b eine erfindungsgemäße Vorrichtung zur Messung eines momentanen Sauerstoffbedarfs pro Zeiteinheit einer Flüssigkeit;

Fig. 3 eine mit der Vorrichtung nach Fig. 2a erhaltene Meßkurve und

Fig. 4 eine Anzeigeeinheit einer erfindungsgemäßen Regeleinrichtung für eine biologische Kläranlage.

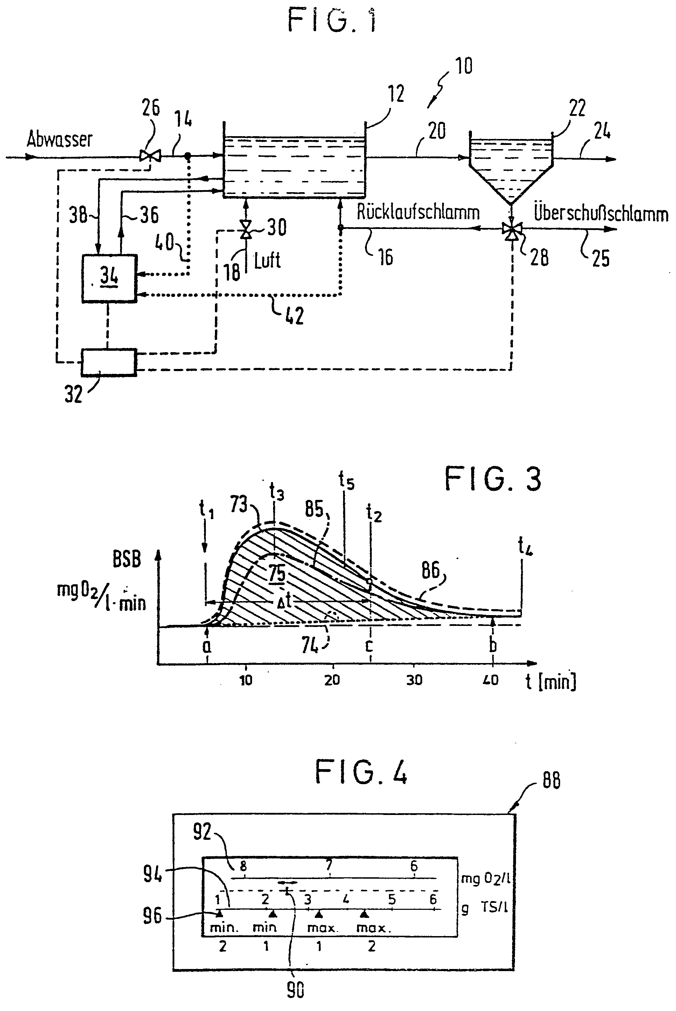

In Fig. 1 ist eine allgemein mit 10 bezeichnete biologische Kläranlage schematisch dargestellt, bei der einem Belebungsbecken 12 das zu reinigende Abwasser über eine Zuleitung 14, Rücklaufschlamm (Belebtschamm) über eine Zuleitung 16 und Luft über eine Zuleitung 18 zugeführt wird. Im Belebungsbecken 12 findet der biologische Abbau der organischen Bestandteile des Abwassers durch die Mikroorganismen des Belebtschlammes statt. Um diese Reaktion zu begünstigen, wird die Flüssigkeit im Belebungsbecken 12 ständig durchmischt und mit Luftsauerstoff versetzt. Das Belebungsbecken 12 wird im kontinuierlichen Durchlaufverfahren betrieben, d.h. das zu reinigende Abwasser wird kontinuierlich über die Zuleitung 14 zugeführt und eine gereinigte Ablaufflüssigkeit über eine Leitung 20 kontinuierlich abgezogen. Durch die entsprechenden Pumpraten und das Volumen des Belebungsbeckens 12 ist die mittlere Verweildauer des Abwassers im Belebungsbecken 12 festgelegt. Sie liegt im praktischen Betrieb etwa zwischen einer halben Stunde und 1 bis 2 Tagen.

Die Ablaufflüssigkeit aus Schlamm und abgebautem Abwasser wird durch die Leitung 20 einer Trenneinrichtung 22 zugeführt, die den Schlamm von der Ablaufflüssigkeit trennt und die so gereinigte Ablaufflüssigkeit über eine Leitung 24 einem Vorfluter zuführt. Als Trenneinrichtung 22 kann man Absetzbecken, Zentrifugen oder dergleichen einsetzen.

Ein Teil des von der Trenneinrichtung 22 abgegebenen Schlammes wird als Rücklaufschlamm durch die Zuleitung 16 dem Belebungsbecken 12 rückgeführt; der andere Teil wird durch eine Leitung 25 als Überschußschlamm von der Kläranlage 10 abgegeben.

Der Betriebsablauf der Kläranlage 10 wird durch Ventile gesteuert, die eine Änderung der Durchflußrate gestatten. Ein erstes Ventil 26 steuert den Abwasserstrom durch die Zuleitung 14. Ein zweites Ventil 28 legt die dem Belebungsbecken 12 durch die Zuleitung 16 zugeführte Rücklaufschlammenge bzw. die über die Leitung 25 abgegebene und dadurch dem Schlammkreislauf entzogene Überschußschlammenge fest. Ein drittes Ventil 30 bestimmt die Luftzufuhrate durch die Zuleitung 18. Daneben können noch weitere Ventile vorgesehen sein, die zur Vereinfachung der Darstellung jedoch nicht gezeigt sind.

Die drei Ventile 26, 28 und 30 werden von einer Regeleinrichtung 32 gesteuert. Mit unterbrochenen Linien sind die elektrischen Leitungen zwischen der Regeleinrichtung 32 und den Ventilen 26, 28 und 30 symbolisiert. Die Regeleinrichtung 32 erhält die Information über den Zustand der Kläranlage 10 von einer Meßeinrichtung 34. Der Meßeinrichtung 34 wird ständig Probensuspension aus dem Belebungsbecken 12 über eine Zuleitung 38 zugeführt und nach der Messung über eine Leitung 36 wieder abgeführt, nachdem in der Meßeinrichtung 34, wie im folgenden noch näher beschrieben, ein momentaner Sauerstoffbedarf pro Zeiteinheit diese Flüssigkeit gemessen wurde. Die Probensuspension besteht, der momentanen Zusammensetzung des Belebungsbekkeninhalts entsprechend, aus Belebtschlamm und mehr oder minder abgebauten organischen Abfallstoffen.

In Fig. 1 sind weitere Zuleitungen 40 und 42 punktiert angedeutet, mit denen eine Steuerung der Kläranlage 10 möglich ist. Hierbei wird nicht der aktuelle Betriebszustand im Belebungsbecken 12 festgestellt und durch entsprechende Regelmaßnahmen auf einem vorgegebenen Normalzustand gehalten, sondern die Zusammensetzung des Abwassers und des Rücklaufschlamms festgestellt, bevor das Abwasser und der Rücklaufschlamm dem Belebungsbecken 12 zugeleitet werden. Durch die Zuleitung 40 wird also eine Abwasserprobe und durch die Zuleitung 42 eine Rücklaufschlammprobe der Meßeinrichtung 34 zugeführt und dort einer Messung des momentanen Sauerstoffbedarfs pro Zeiteinheit unterzogen. Je nach Zusammensetzung des Abwassers und des Rücklaufschlammes werden dann die Ventile 26, 28 und 30 eingestellt.

Die Kläranlage 10 kann auch mit einem Verfahren betrieben werden, welches des Regelverfahren mit dem Steuerverfahren kombiniert.

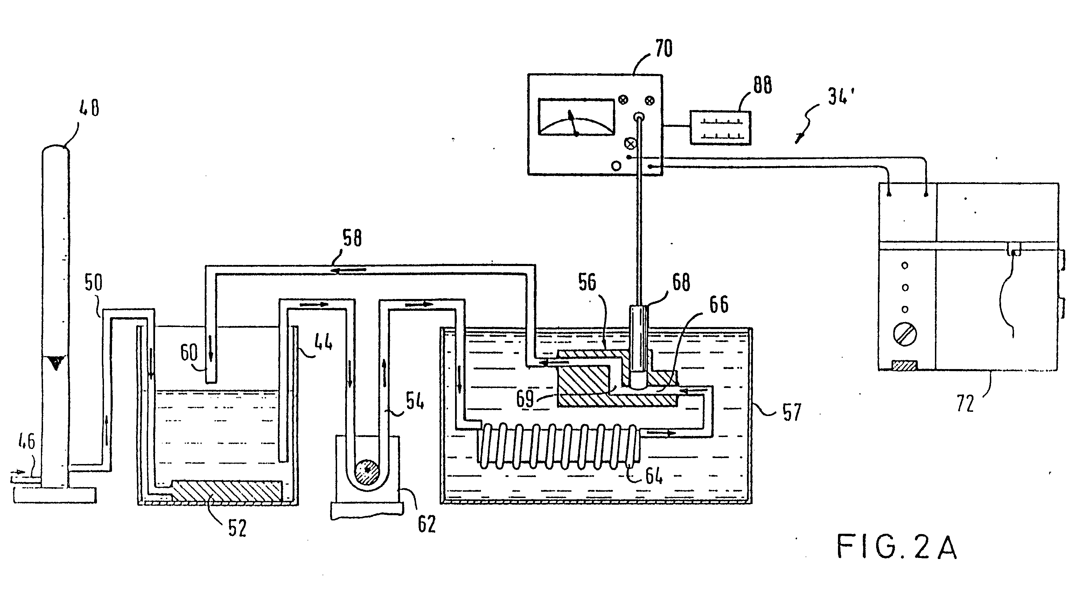

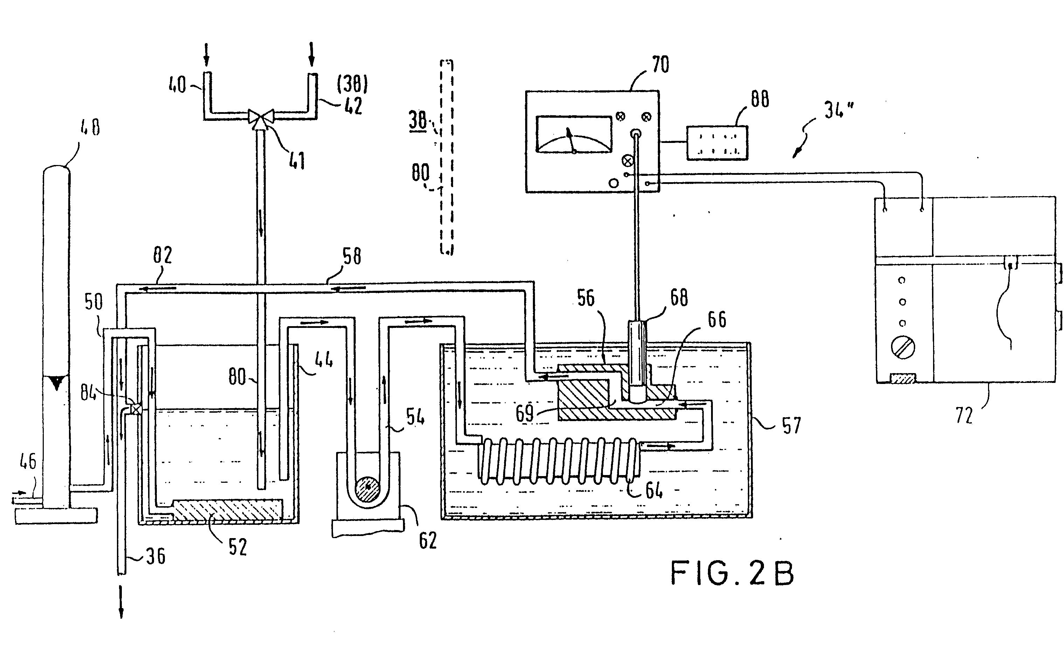

Die Fig. 2a und 2b zeigen zwei Ausführungsformen 34' und 34'' der Meßeinrichtung 34, die unter anderem auch zur Steuerung und Regelung der in Fig. 1 dargestellten Kläranlage 10 verwendet werden können. Bauteile, die in beiden Ausführungsformen 34' und 34'' gleich sind, tragen die gleichen Bezugsziffern. So ist jeweils ein Reaktionsbehälter 44 vorgesehen, in dem sich die zu untersuchende Belebtschlamm-Abwasser-Suspension befindet. Sie wird von einer Belüftungseinrichtung derart mit Luft versorgt, daß sich ein Sauerstoffsättigungswert in der Suspension einstellt. Die Belüftungseinrichtung besteht aus einer nicht gezeigten Luftpumpe, die durch eine Zuleitung 46 Luft einem Rotameter 48 zuführt, der für konstante Luftzuführrate sorgt. Vom Rotameter 48 führt eine Leitung 50 zu einem Begasungsstein 52 an der Bodeninnenseite des Reaktionsbehälters 44, der für einen feinverteilten Gasaustritt sorgt. Es werden etwa 100 Liter Luft pro h zugeführt. (Die Sauerstoffzufuhr kann auch durch Zugabe von $H_2O_2$ erfolgen). Der Reaktionsbehälter 44 nimmt etwa 3 Liter Suspension auf, die von einem Thermostaten auf konstante Temperatur von 20°C gehalten wird und die von einer nicht gezeigten Rühreinrichtung ständig durchmischt wird.

Aus dem Reaktionsbehälter 44 führt jeweils eine (nach aussen hin abgeschlossene) Zuleitung 54 zu einer Meßzelle 56 in einem Wasserbadbehälter 57; in der Ausführungsform 34' führen von der Meßzelle 56 weitere Leitungen 58 und 60 zurück zum Reaktionsbehälter 44, wodurch ein geschlossener Kreislauf gebildet wird. In der Ausführungsform 34'' dagegen setzt sich die Leitung 58 in einer Ableitung 82 fort, die die Flüssigkeit (Suspension) von der Meßeinrichtung 34'' wegführt. In die Ableitung 82 mündet ein Überlauf 84, der den Pegel im Reaktionsbehälter konstant hält. Dies ist hier notwendig, da, wie noch zu behandeln, dem Reaktionsbehälter 44 ständig Probensuspension über eine Leitung 80 zugeführt wird.

Die Zuleitung 54 führt jeweils durch eine schematisch dargestellte Schlauchpumpe 62, die die Flüssigkeit mit etwa 2 Litern pro min durch die Zuleitung 54 pumpt. Die resultierende hohe Fließgeschwindigkeit sorgt dafür, daß sich keine Ablagerungen in den Leitungen 54, 58 und 60 und in der Meßzelle 56 bilden. Sie beträgt etwa 125 cm pro s. Um eine ausreichend große Laufzeit zwischen Reaktionsbehälter 44 und Meßzelle 56 zu erhalten, hat die Zuleitung 54 eine Länge von etwa 6 m, wobei 5 m davon als Schlauchspirale 64 auf einen Wickelkörper gewickelt sind. Das Wasserbad innerhalb des Wasserbadbehälters 57 wird thermostatisch auf 20°C gehalten.

Die Aufteilung der Meßeinrichtungen 34' und 34'' jeweils in zwei thermostatisierte Behälter 44, 57 bietet den Vorteil, daß das unter möglichst idealen Reaktionsbedingungen zu haltende Reaktionsvolumen klein gehalten werden kann, da die viel Raum benötigenden Bauteile Meßzelle 56 und Zuleitungen 54, 58, 60 anderweitig (im Wasserbadbehälter 57) untergebracht sind.

In den die jeweilige Meßzelle 56 durchsetzenden Durchflußkanal 66 ragt eine $O_2$-Meßzelle 68. Diese gibt an eine Verstärker- und Anzeigeeinheit 70 ein elektrisches Signal ab, welches die momentane Sauerstoffkonzentration der Suspension im Durchflußkanal 66 darstellt. An die Einheit 70 kann ein Schreiber 72 (x,t-Schreiber) angeschlossen werde, der den Zeitverlauf des von der Meßzelle 68 abgegebenen Signals, d.h. der Sauerstoffkonzentration im Durchflußkanal 66 aufzeichnet.

Der Durchflußkanal 66 ist in Strömungsrichtung unmittelbar anschließend an die Meßzelle 68 geknickt, da die im Bereich des Knicks 69 induzierten Wirbel eine Schlammablagerung im Bereich der Meßzelle 68 und die Bildung von Luftblasen an dieser Stelle wirksam verhindern.

Mit den Meßeinrichtungen 34' und 34'' läßt sich der momentane Sauerstoffbedarf der im Reaktionsbehälter 44 befindlichen Suspension messen. Im Reaktionsbehälter 44 ist der Anteil des in der Suspension gelösten Sauerstoffs auf dem Sättigungswert. Während der Laufzeit durch die Zuleitung 54 nimmt der Sauerstoffgehalt jedoch ab, da die Mikroorganismen weiterhin Sauerstoff verbrauchen, neuer Sauerstoff jedoch nicht nachgeliefert wird. Dieser Sauerstoffverbrauch ist abhängig von der Zusammensetzung der Suspension, d.h. er hängt von der Art und Menge der organischen Abfallstoffe und der Mikroorganismen ab. Die Meßzelle 68 wird daher eine Sauerstoffkonzentration messen, die geringer ist als die im Reaktionsbehälter 44 herrschende Sättigungskonzentration. Aus der Differenz der Konzentrationen und dem Volumendurchsatz durch die Meßzelle 68 läßt sich daher der momentane biologische Sauerstoffbedarf der Suspension im Reaktionsbehälter 44 berechnen, beispielsweise mit der Einheit mg Sauerstoff pro Liter und min.

Fig. 3 zeigt eine Meßkurve der Meßeinrichtung 34', bei der die Abszisse die Zeitachse darstellt und die Ordinate die bereits in Sauerstoffverbrauchswerte umgerechneten, mit BSB symbolisierten Signalwerte darstellt. Steigender Sauerstoffverbrauch und damit wachsendem Konzentrationsabfall in der Zuleitung 54 entspricht fallende Meßspannung an der Meßzelle 68.

Am Versuchsbeginn (z.Z. t = 0) befindet sich im Reaktionsbehälter 44 eine Belebtschlammsuspension mit etwa 3—8 g, vorzugsweise 6 g, Trockensubstanz pro Liter. Es stellt sich ein momentaner Sauerstoffverbrauch pro Zeiteinheit a ein, der die endogene oder Grund-Atmung des Belebtschlamms kennzeichnet. Zum Zeitpunkt $t_1$ wird eine organische Substanz beispielsweise Abwasser, zugefügt, so daß sich im Reaktionsbehälter 44 ein BSB-Wert von etwa 33 mg pro Liter einstellt. Daraufhin steigt der pro Zeiteinheit benötigte Sauerstoffbedarf entsprechend der Kurve 73 in Fig. 3 an, da die Mikroorganismen zum Abbau zusätzlichen Sauerstoff benötigen. Nach erfolgtem Abbau innerhalb von 15—60 min, je nach Art der beteiligten Substanzen, stellt sich wieder eine konstante Sauerstoffaufnahmerate b ein, die etwa gleich der Aufnahmerate a zu Beginn der Messung ist. Die Meßkurve 73 stellt also eine Abbaukurve dar, die für das Belebtschlamm-Abwassersystem charakteristisch ist. Aus der Fläche 75, die durch die Meßkurve 73 und eine die Endwerte a und b verbindende Gerade 74 begrenzt ist, läßt sich durch Multiplikation mit einem einmal ermittelten, vom Versuchsaufbau (Länge der Zuleitung 54, Pumpleistung der Schlauchpumpe 62, Sauerstoffsättigungswert, Eichung und Empfindlichkeit der $O_2$-Meßzelle 68) abhängigen ersten Faktor der anfangs genannte Kurzzeit-BSB-Wert berechnen. Bei bekannten Abwasserverhältnissen kann aus dem Kurzzeit-BSB-Wert durch Multiplikation mit einem einmal ermittelten zweiten Faktor der $BSB_5$-Wert errechnet werden.

Mit der Meßeinrichtung 34' läßt sich also die Abbaukurve 73 einer einzelnen zur Zeit $t_1$ zugefügten Substratprobe messen. Man kann jedoch auch auf die Messung des Gesamtverlaufs der Kurve 73 verzichten und dafür den momentanen Sauerstoffbedarf pro Zeiteinheit eines kontinuierlichen Stroms aus Substrat und Belebtschlamm zu einem Zeitpunkt $t_2$ messen, der um eine Reaktionsdauer $\Delta t$ später ist, als der Zeitpunkt $t_1$ der Zugabe der Probe (siehe Fig. 3). Um diese Reaktionsdauer $\Delta t$ zu erhalten, ist die Meßeinrichtung in der zweiten Ausführungsform 34'' so ausgebildet, daß der Reaktionsbehälter 44 als Durchlaufbehälter dient mit einer mittleren Verweildauer der Suspension im Reaktionsbehälter 44, die gleich der Reaktionsdauer $\Delta t$ ist. In dieser Ausführungsform 34'' wird dem Reaktionsbehälter 44 über eine Zuleitung 80 kontinuierlich Probensuspension zugeführt. Nach der Messung in der Meßzelle 68 wird die Probensuspension durch die Leitung 82 abgeführt. Die Volumenrate, mit der im Reaktionsbehälter 44 durch die Zuleitung 80 Probensuspension zugeführt wird, ist in Abhängigkeit vom Suspensionsvolumen im Reaktionsbehälter 44 derart festgelegt, daß die mittlere Verweildauer der vorgegebenen Reaktionsdauer $\Delta t$ entspricht. Solange sich die Zusammensetzung der zu messenden Suspension nicht ändert, erhält man ein konstantes Meßsignal, dessen dem Wert c entsprechende Größe durch $\Delta t$ festgelegt ist. Verändert sich jedoch die Zusammensetzung, ergeben sich unterschiedliche Signaländerungen in Abhängigkeit von $\Delta t$. Verringert sich beispielsweise die Konzentration der organischen Substanzen, so würde man mit der ersten Ausführungsform 34' eine Meßkurve 85 (siehe Fig. 3) erhalten. Ist $\Delta t$ so festgelegt, daß die Messung mit der zweiten Ausführungsform 34'' am Kurvenmaximum zur Zeit $t_3$ stattfindet, so ist die Meßwertänderung maximal. Dagegen würde eine Messung zum Zeitpunkt $t_4$ weiterhin einen konstanten Meßwert liefern. Eine Messung der Substratkonzentration wird daher zweckmäßigerweise zum Zeitpunkt $t_3$ durchgeführt. Eine Änderung des TS-Gehalts, beispielsweise eine Vergrößerung des Belebtschlammanteils, würde zu einer geringfügig nach oben verschobenen Meßkurve 86 führen. Die Messung des TS-Gehaltes mit der Ausführungsform 34'' sollte daher zum Zeitpunkt $t_4$ stattfinden, da eine Meßwertänderung zu diesem Zeitpunkt ausschließlich auf eine TS-Gehalt-Veränderung schließen läßt.

Wenn man einen Meßzeitpunkt $t_5$ wählt, der im auslaufenden Zweig der Meßkurven 73, 85 und 86 liegt, vorzugsweise auf halber Maximalhöhe, kann man die Steuerung oder Regelung so vornehmen, daß man bei größeren Signaländerungen auf eine Änderung der Substratkonzentration oder auf toxische Substanzen im Substrat schließt und bei geringeren, insbesondere sich nur langsam einstellenden Änderungen auf eine TS-Gehalt-Änderung schließt.

Wird die Meßeinrichtung 34'' zur Steuerung der Kläranlage 10 eingesetzt, so ist die Zuleitung 80 an ein Dosierventil 41 angeschlossen, dem über die Leitungen 40 und 42 Abwasser bzw. Belebtschlamm zufließt, und das an die Leitung 80 Probensuspension aus Abwasser und Schlamm mit vorgegebenem Volumenverhältnis abgibt; die Leitung 82 ist an die in das Belebungsbecken 12 rückführende Leitung 36 angeschlossen. Der Belebtschlammanteil der in den Reaktionsbehälter 44 fließenden Probensuspension ist folglich annähernd konstant. Die Messung wird zur Zeit $t_3$ durchgeführt; Meßwertänderungen sind direkt auf Änderungen der Substratzusammensetzung bzw. des Substratgehalts oder auf toxische Stoffe zurückführbar.

Wenn man anstelle der Rückführschlamm führenden Leitung 42 die an das Belebungsbecken 12 angeschlossene Leitung 38 an das Dosierventil 41 anschließt (in Fig. 2b in Klammern angedeutet), so erhält man ebenfalls Meßwerte, die einen momentanen biologischen Sauerstoffbedarf angeben, da auch in der Flüssigkeit des Belebungsbeckens 12 Belebtschlamm, wenn auch in vergleichsweise geringerer Konzentration, vorhanden ist. Die Meßwerte sind vom TS-Gehalt der Belebungsbeckenflüssigkeit abhängig, so daß auch der TS-Gehalt mit Hilfe dieser Meßwerte kontrolliert werden kann.

In der in Verbindung mit der Fig. 1 bereits beschriebenen Regelanordnung der Kläranlage 10 wird der Meßeinrichtung 34'' über die Leitung 38 und die daran angeschlossene Leitung 80 (in Fig. 2b mit unterbrochenen Linien zusätzlich dargestellt) Probensuspension aus dem Belebungsbecken 12 zugeführt. Dem Abbaugrad und der Durchmischung der Flüssigkeit im Belebungsbecken 12 entsprechend enthält die Probensuspension Belebtschlamm und mehr oder minder abgebaute Abfallstoffe, so daß sich bei einer entsprechenden Messung mit der ersten Ausführungsform 34'' der Meßvorrichtung eine relativ flache, mit wachsendem t auf einen konstanten Wert fallende Meßkurve ergibt, die eine Superposition von zeitverschobenen Meßkurven 73 der Fig. 3 darstellt. Der genannten Meßkurve lassen sich ebenfalls Zeitpunkte $t_3$, $t_4$ und $t_5$ entsprechend Fig. 3 zuordnen; die voranstehenden Überlegungen zur Wahl des Zeitpunktes der Messung mit der zweiten Ausführungsform 34'' der Meßvorrichtung gelten entsprechend.

In Fig. 4 ist die Anzeigeskala einer Anzeigeeinheit 88 dargestellt, die an die Regel- und Meßeinrichtungen 32 und 34 der Kläranlage 10 bzw. an die Verstärker- und Anzeigeeinheit 70 anzuschließen ist. Es ist hier ein Meßwertanzeiger 90 dargestellt, der als Zeiger, als Leuchtpunkt oder als Kurvenschreiber ausgeführt sein kann und der in Pfeilrichtung beweglich ist. Oberhalb des Meßwertanzeigers 90 ist eine erste Skala 92 angebracht, die die Sauerstoffkonzentration in der Meßzelle 68 anzeigt. Unterhalb des Meßwertanzeigers 90 ist eine zweite Skala 94 angebracht, bei der aus den Werten der ersten Skala 92 TS-Gehalt-Werte berechnet sind, unter der Annahme, daß die Messung nur die endogene Atmung erfaßt.

An der zweiten Skala 94 sind Schaltmarken 96 angebracht, die mit max. 1, max. 2, min. 1 und min. 2 bezeichnet sind. Diese Schaltmarken 96 schalten, sobald der Meßwertanzeiger 90 an ihnen vorbeiläuft, woraufhin die Kläranlage 10 nachgesteuert wird, mit dem Ziel der Wiedereinstellung der Soll-Lage des Meßwertanzeigers 90 zwischen den Marken max. 1 und min. 1. Um sowohl den TS-Gehalt als auch den Substratgehalt überwachen und steuern bzw. regeln zu können und um rechtzeitig den Zufluß toxischer Stoffe bemerken und weiteren Zufluß dieser Stoffe verhindern zu können, wird die Messung etwa zum Zeitpunkt $t_5$ durchgeführt. Sobald der Meßwertanzeiger 90 den min.1-Kontakt unterschreitet, wird über das Ventil 28 die Abzugsrate des Überschußschlamms aus dem Schlammkreislauf reduziert, da aus dem Meßsignal auf einen verringerten TS-Gehalt geschlossen wird. Bewegt sich der Meßwertanzeiger 90 jedoch weiter und unterschreitet er die min.2-Marke, so wird über das Ventil 26 die Abwasserzuführrate stark gedrosselt und ein Alarm ausgelöst, da jetzt auf eine Vergiftung der Flüssigkeit im Belebungsbecken 12 durch das Abwasser geschlossen wird. Wird die max.1-Marke überfahren, so wird über das Ventil 28 die Überschußschlammabzugsrate erhöht, um den TS-Gehalt herabzusetzen. Wird die max.2-Marke überschritten, so wird über das Ventil 26 der Abwasserzulauf reduziert, da aus den Meßsignalen auf einen erhöhten organischen Schmutzanteil im Abwasser geschlossen wird. Zusätzlich kann die Luftzufuhr über das Ventil 30 oder über das Zuschalten eines weiteren Belüftungsaggregats erhöht werden, um den Abbau der zusätzlichen Schmutzstoffe zu erleichtern; auch kann in diesem Fall dem Belebungsbecken 12 zusätzlicher Belebtschlamm zugeführt werden. Zusätzlich wird ein Alarm ausgelöst.

Anstelle der eben beschriebenen Messung zum Zeitpunkt $t_5$, die eine Änderung sowohl des TS-Gehalts als auch des Substratgehalts bzw. des Gehalts an toxischen Inhaltsstoffen erfaßt, kann durch Änderung der Reaktionsdauer $\Delta t$ im Reaktionsbehälter 44 hintereinander eine Messung des TS-Gehalts (Zeitpunkt $t_4$) und eine Messung des Substratgehalts bzw. der Toxizität (Zeitpunkt $t_3$) durchgeführt werden. Bei der Messung des TS-Gehalts wird bei Überfahren der angesprochenen Marken min. 1 und max. 1, wie bereits angeführt, die Überschußschlammabzugesrate geregelt oder gesteuert, während bei der Messung des Substratgehaltes bzw. der Toxizität und überfahren der Marken min. 2 und max. 2 die Substratzuführrate geregelt oder gesteuert wird.

Die bei Überschreiten der Schaltmarken 96 eingeleiteten Maßnahmen werden entweder nach einer vorbestimmten Zeit wieder rückgängig gemacht oder nachdem der Meßwertanzeiger 90 die entsprechende Schaltmarke 96 wieder in entgegengesetzter Richtung überfährt.

Die zweite Skala 94 ist auswechselbar, so daß bei einer Änderung der Art des Belebtschlammes eine entsprechend andere Skala 94 eingebaut werden kann.

**Patentansprüche**

1. Verfahren zur Regelung und/oder Steuerung einer biologischen Kläranlage mit einem Belebungsbecken, dem kontinuierlich Abwasser und Belebtschlamm zugeführt und aus dem Ablaufflüssigkeit abgezogen wird, und mit einer die Ablaufflüssigkeit aufnehmenden Trenneinrichtung, die den Schlamm von der Ablaufflüssigkeit trennt und in das Belebungsbecken teilweise rückführt, wobei man einen biologischen Sauerstoffbedarfswert einer Belebungsbeckenflüssigkeit enthaltenden Probenflüssigkeit mißt und der Regelung und/oder Steuerung der Kläranlage zugrundelegt, dadurch gekennzeichnet, daß man zur Regelung des TS-Gehalts im Belebungsbecken dem Belebungsbecken kontinuierlich Belebungsbeckenflüssigkeit zur Bildung der Probenflüssigkeit entnimmt und daß man die Probenflüssigkeit während einer vorgegebenen Reaktionsdauer $\Delta t$ mit Sauerstoff im Überschuß versetzt und anschließend den momentanen biologischen Sauerstoffbedarf pro Zeiteinheit der Probenflüssigkeit kontinuierlich mißt, wobei man die Reaktionsdauer $\Delta t$ derart festlegt, daß der biologische Abbau des Abwassers in der Probenflüssigkeit innerhalb der Reaktionsdauer im wesentlichen beendet ist.

2. Verfahren nach Anspruch 1, dadurch gekennzeichnet, daß die Reaktionsdauer $\Delta t$ 1 bis 60 min, besser 18 bis 30 min, am besten 20 min beträgt.

3. Verfahren nach Anspruch 1, dadurch gekennzeichnet, daß man eine weitere derartige Messung vornimmt, bei der man die Reaktionsdauer $\Delta t$ jedoch derart festlegt, daß am Ende der Reaktionsdauer $\Delta t$ die Substratatmungsrate der Probenflüssigkeit im wesentlichen maximal ist.

4. Verfahren nach Anspruch 3, dadurch gekennzeichnet, daß die Reaktionsdauer $\Delta t$ 1 bis 20 min, besser 2 bis 10 min, am besten 5 min beträgt.

5. Verfahren nach Anspruch 1, dadurch gekennzeichnet, daß die Messung dann durchgeführt wird, wenn die Substratatmungsrate auf einen Wert abgefallen ist, der 1/5 bis 4/5, besser 2/5 bis 3/5, am besten der Hälfte der maximalen Substratatmungsrate entspricht.

6. Verfahren nach einem der Ansprüche 1 bis 5, dadurch gekennzeichnet, daß man in einer weiteren Messung mit dem dem Belebungsbecken kontinuierlich zufließenden Abwasser kontinuierlich Probenflüssigkeit bildet und den biologischen Sauerstoffbedarf pro Zeiteinheit kontinuierlich mißt.

7. Verfahren nach einem der Ansprüche 1 bis 6, dadurch gekennzeichnet, daß die Rückführrate des Belebtschlamms in Abhängigkeit vom gemessenen Sauerstoffbedarf pro Zeiteinheit eingestellt wird.

8. Verfahren nach einem der Ansprüche 1 bis 7, dadurch gekennzeichnet, daß die Abführrate des Überschußschlamms in Abhängigkeit vom gemessenen Sauerstoffbedarf pro Zeiteinheit eingestellt wird.

9. Verfahren nach einem der Ansprüche 1 bis 8, dadurch gekennzeichnet, daß die Abwasserzuführrate in Abhängigkeit vom gemessenen Sauerstoffbedarf pro Zeiteinheit eingestellt wird.

10. Verfahren nach einem der Ansprüche 1 bis 9, dadurch gekennzeichnet, daß dem Belebungsbecken Sauerstoff zugeführt wird mit einer vom gemessenen Sauerstoffbedarf pro Zeiteinheit abhängigen Zuführrate.

11. Verfahren nach einem der Ansprüche 1 bis 10, dadurch gekennzeichnet, daß die Abführrate des Überschußschlammes erhöht wird, wenn der gemessene Sauerstoffbedarf einen vorgegebenen ersten Maximalwert (max. 1) überschreitet und daß die Abführrate erniedrigt wird, wenn der gemessene Sauerstoffbedarf einen vorgegebenen ersten Minimalwert (min. 1) unterschreitet.

12. Verfahren nach Ansprüch 11, dadurch gekennzeichnet, daß die Abwasserzuführrate verringert wird, wenn der gemessene Sauerstoffbedarf einen vorgegebenen zweiten Maximalwert (max. 2) überschreitet und daß die Abwasserzuführrate weiter verringert wird, wenn der gemessene Sauerstoffbedarf einen vorgegebenen zweiten Minimalwert (min. 2) unterschreitet, wobei der zweite Maximalwert (max. 2) größer als der erste Maximalwert (max. 1) und der zweite Minimalwert (min. 2) kleiner als der erste Minimalwert (min. 1) ist.

13. Verfahren nach Anspruch 11 oder 12, dadurch gekennzeichnet, daß die Änderung der Abwasser- und Überschußschlammzuführraten nach Ablauf vorgegebener Zeitintervalle rückgängig gemacht wird.

14. Verfahren nach einem der Ansprüche 1 bis 13, dadurch gekennzeichnet, daß man die Probenflüssigkeit bei der kontinuierlichen Messung des momentanen biologischen Sauerstoffbedarfs durch eine nach außen hin abgeschlossen Zuleitung und einen sich anschließenden Durchflußkanal einer Meßzelle mit einer Fließgeschwindigkeit von 50 bis 200 cm/sec, vorzugsweise 110 bis 140 cm/sec, am besten 125 cm/sec, pumpt.

15. Vorrichtung zur Durchführung des Verfahrens nach einem der Ansprüche 1 bis 14, mit einem die Probenflüssigkeit aufnehmenden Reaktionsbehälter, einer den Sauerstoffgehalt der Flüssigkeit im Reaktionsbehälter auf dem Sättigungswert haltenden Belüftungseinrichtung, einer mit einem Durchflußkanal versehenen Meßzelle zur Messung des Sauerstoffgehalts, einer nach außen hin abgeschlossenen, von Ablagerungen frei zu haltende Zuleitung zwischen dem Reaktionsbehälter und der Meßzelle und mit einer Pumpe, die Flüssigkeit durch die Zuleitung zur Meßzelle pumpt, dadurch gekennzeichnet, daß die Zuleitung (54) eine Länge von 2 bis 10 m, vorzugsweise 4 bis 8 m, am besten 6 m, aufweist.

16. Vorrichtung nach Anspruch 15, dadurch gekennzeichnet, daß der Durchflußkanal (66) der Meßzelle (68) in Strömungsrichtung anschließend an die meßzelle 68 einen Knick (69) aufweist.

## Claims

1. Process for the regulation and/or control of a biological clarification plant comprising an activation tank, into which effluent and activation slurry is continuously poured and from which the discharge liquid is drawn off and a separation means, which accepts said discharge liquid, separating the slurry from the discharge liquid and partially returns it to the activation tank, whereby a biological oxygen requirement value of a sample liquid containing the activation tank liquid is measured and is used as the basis for the regulation and/or control of the clarification plant, characterized in that to regulate the TS content in the activation tank, activation tank liquid is continuously drawn off from said activation tank to form the sample liquid, and that the sample liquid has excess oxygen added to it during a predetermined reaction time t and then the momentary biological oxygen requirement is continuously measured per time unit of the sample liquid, whereby the reaction time t is fixed in such a manner that the biological decomposition of the effluent in the sample liquid is substantially ended within said reaction time.

2. Process according to claim 1, in which the reaction time $\Delta t$ extends from 1 to 60 minutes, preferably from 18 to 30 min, and optimally 20 min.

3. Process according to claim 1, such that a further measurement of this type is made, in which the reaction time $\Delta t$ is however so determined that at the end of said reaction time $\Delta t$ the substrate respiration rate of the sample liquid is substantially maximalized.

4. Process according to claim 3, wherein the reaction time $\Delta t$ is 1 to 20 min, preferably 2 to 10 min, and optimally 5 min.

5. Process according to claim 1, wherein the measurement is taken when the substrate respiration rate has fallen to a value which corresponds to 1/5 to 4/5, preferably 2/5 to 3/5 and optimally to half the maximal substrate respiration rate.

6. Process according to one of the claims 1 to 5 above, wherein in a further measurement, sample liquid is continuously formed using the effluent constantly flowing into the activation tank and the biological oxygen requirement per unit of time is continuously measured.

7. Process according to one of the claims 1 to 6 above, wherein the reflux rate of the activated slurry is adjusted depending on the measured oxygen requirement per unit of time.

8. Process according to one of the claims 1 to 7 above, wherein the discharge rate of the excess slurry is adjusted depending on the measured oxygen requirement per unit of time.

9. Process according to one of the claims 1 to 8 above, wherein the effluent supply rate is adjusted depending on the measured oxygen requirement per unit of time.

10. Process according to one of the claims 1 to 9 above, wherein oxygen is supplied to the activation tank at a supply rate which is dependent on the measured oxygen requirement per unit of time.

11. Process according to one of claims 1 to 10 above, wherein the discharge rate of the excess slurry is increased when the measured oxygen requirement exceeds a predetermined first maximal value (max.1) and wherein the discharge rate is reduced, when the measured oxygen requirement falls below a predetermined first minimal value (min.1).

12. Process according to claim 11 above, characterized in that the effluent supply rate is reduced when the measured oxygen rate exceeds a predetermined maximal value (max.2) and that the effluent supply rate is still further reduced when the measured oxygen requirement falls below a second minimal predetermined value (min.2) whereby the second maximal value (max.2) is larger than the first maximal value (max.1) and the second minimal value (min.2) is smaller than the first minimal value (min.1).

13. Process according to claim 11 or 12, wherein the change in the effluent- and excess slurry-supply rates is cancelled after the expiry of a predetermined period of time.

14. Process according to one of the claims 1 to 13 above, wherein the sample liquid, during the continuous measurement of the momentary biological oxygen requirement, is pumped through a pipe sealed off from the outside and via a flow channel of a measuring cell connected therewith at a flow speed of 50 to 200 cm/sec, preferably at 110 to 140 cm/sec, and optimally at 125 cm/sec.

15. Device for the completion of the process according to claims 1 to 14 above, comprising a reaction container to hold the sample liquid, a ventilation device to keep the oxygen content of the liquid in said reaction container at the saturation value, a measuring cell equipped with a flow channel to measure the oxygen content, a pipe sealed off from the outside which is kept free of deposits between said reaction container and said measuring cell, and a pump which pumps liquid through the pipe to the measuring cell, wherein said pipe (54) has a length of 2 to 10 metres, preferably 4 to 8 m, and optimally 6 m.

16. Device according to claim 15, wherein the flow channel (66) of the measuring cell (68) has a sharp bend (69) in the direction of flow adjacent to the measuring cell (68).

**Revendications**

1. Procédé pour le réglage et/ou la commande d'une installation d'épuration biologique avec un bassin d'aération, auquel on amène de façon continue de l'eau résiduaire et de la boue activée et duquel on soutire du liquide de décharge, et avec un dispositif séparateur reprenant le liquide de décharge qui sépare la boue du liquide de décharge et la renvoie partiellement dans le bassin d'aération, procédé dans lequel on mesure la valeur de la demande d'oxygène biologique d'un liquide-échantillon contenant du liquide du bassin d'aération et on se base sur cette valeur pour le réglage et/ou la commande de l'installation d'épuration, caractérisé en ce qu'on soutire continuellement, pour le réglage de la teneur en M.e.S. dans le bassin d'aération, de la liqueur de bassin d'aération dans le bassin d'aération pour la formation d'un liquide-échantillon, en ce qu'on a ajoute au liquide-échantillon pendant une durée réactionnelle fixée à l'avance Δt de l'oxygène en excès et en ce qu'ensuite on mesure continuellement la demande d'oxygène biologique instantanée par unité de temps du liquide-échantillon, en fixant la durée réactionnelle Δt de telle sorte que la dégradation biologique de l'eau résiduaire est essentiellement terminée dans le liquide-échantillon en-deçà de la durée réactionnelle.

2. Procédé selon la revendication 1, caractérisé en ce que la durée réactionnelle Δt est de 1 à 60 minutes, mieux 18 à 30 minutes, le mieux 20 minutes.

3. Procédé selon la revendication 1, caractérisé en ce qu'on entreprend une mesure semblable supplémentaire, dans laquelle toutefois on fixe la durée réactionnelle Δt de telle sorte qu'à la fin de la durée réactionnelle Δt, la part de respiration du substrat du liquide-échantillon soit essentiellement maximale.

4. Procédé selon la revendication 3, caractérisé en ce que la durée réactionnelle Δt est de 1 à 20 minutes, mieux 2 à 10 minutes, de préférence à 5 minutes.

5. Procédé selon la revendication 1, caractérisé en ce que l'on réalise la mesure au moment où la part de respiration du substrat est tombée à une valeur qui est de 1/5 à 4/5, mieux 2/5 à 3/5, au mieux de la moitié de la part maximale de respiration du substrat.

6. Procédé selon l'une quelconque des revendications 1 à 5, caractérisé en ce que, dans une mesure supplémentaire, on forme avec l'eau résiduaire d'écoulant continuellement dans le bassin d'aération un liquide-échantillon et qu'on mesure de façon continue la demande biologique en oxygène par unité de temps.

7. Procédé selon l'une quelconque des revendications 1 à 6, caractérisé en ce que l'on fixe la part de recyclage de la boue activée en fonction de la demande en oxygène par unité de temps qui a été mesurée.

8. Procédé selon l'une quelconque des revendications 1 à 7, caractérisé en ce que l'on fixe la part d'élmination de la boue en excès en fonction de la demande en oxygène par unité de temps qui a été mesurée.

9. Procédé selon l'une quelconque des revendications 1 à 8, caractérisé en ce que l'on fixe la part d'eau résiduaire amenée en fonction de la demande en oxygène par unité de temps qui a été mesurée.

10. Procédé selon l'une quelconque des revendications 1 à 9, caractérisé en ce que l'on amène de l'oxygène dans le bassin d'aération en une quantité dépendant de la demande d'oxygène par unité de temps qui a été mesurée.

11. Procédé selon l'une quelconque des revendications 1 à 10, caractérisé en ce que l'on augmente la part de la boue en excès enlevée quand la demande en oxygène mesurée dépasse une première valeur maximale fixée au préalable (max. 1) et en ce que l'on diminue la part de la boue en excès enlevée quand la demande en oxygène mesurée passe en dessous d'une première valeur minimale (min. 1) fixée à l'avance.

12. Procédé selon la revendication 11, caractérisé en ce que l'on diminue la quantité d'eau résiduaire amenée quand la demande en oxygène mesurée dépasse une deuxième valeur maximale (max. 2) fixée à l'avance et en ce que l'on diminue encore la quantité d'eau résiduaire amenée quand la demande en oxygène mesurée passe en dessous d'une deuxième valeur minimale (min. 2) fixée à l'avance, la deuxième valeur maximale (max. 2) étant plus grande que la première valeur maximale (max. 1) et la deuxième valeur minimale (min. 2) plus petite que la première valeur minimale (min. 1).

13. Procédé selon la revendication 11 ou 12, caractérisé en ce que l'on inverse le changement des quantités d'eau résiduaire et de boue en excès amenées après l'écoulement d'un intervalle de temps fixé à l'avance.

14. Procédé selon l'une quelconque des revendications 1 à 13, caractérisé en ce que l'on pompe le liquide-échantillon avec mesure continuelle de la demande biologique d'oxygène instantanée dans une conduite d'arrivée fermée vers l'extérieur et un canal de passage d'une cellule de mesure relié à la conduite avec une vitesse d'écoulement de 50 à 200 cm/sec, de préférence 110 à 140 cm/sec, le mieux 125 cm/sec.

15. Dispositif pour la mise en oeuvre du procédé selon l'une quelconque des revendications 1 à 14 comprenant un récipient réactionnel recevant le liquide-échantillon, un dispositif d'aération maintenant la teneur en oxygène du liquide dans le récipient réactionnel à la valeur de saturation, une cellule de mesure munie d'un canal de passage pour mesurer la teneur en oxygène, une conduite d'amenée fermée à l'extérieur et devant être maintenue dépourvue de dépôt entre le récipient réactionnel et la cellule de mesure, et une pompe qui pompe le liquide dans la conduite d'amenée vers la cellule de mesure, caractérisé en ce que la conduite d'amenée (54) présente une longueur de 2 à 10 m, de préférence 4 à 8 m, le mieux 6 m.

16. Dispositif selon la revendication 15, caractérisé en ce que le canal de passage (66) de la cellule de mesure (68) présente, à la suite de la cellule de mesure (68) dans le sens du courant, un coude (69).

0 009 580

## FIG. 1

## FIG. 3

## FIG. 4

1

FIG. 2A

0009 580

FIG. 2B

0 009 580